# EUROPEAN PATENT APPLICATION

(11) **EP 4 563 592 A1**
(43) Date of publication of application: **04.06.2025**
(21) Application number: 23845678.4
(22) Date of filing: 28.07.2023
(51) Int. Cl.: C07K 14/39, C07K 19/00, C12N 15/62, C12N 15/81, C12N 1/19, C12R 1/84

(54) **CHIMERIC TRANSCRIPTIONAL ACTIVATOR AND USE THEREOF**

(30) Priority: 29.07.2022 CN 202210910746
(71) Applicant: Nanjing Bestzyme Bio-engineering Co. Ltd., Nanjing, Jiangsu 211100 (CN)
(72) Inventor: LIN, Jie, Nanjing, Jiangsu 211100 (CN); BAI, Aixi, Nanjing, Jiangsu 211100 (CN); ZHENG, Fei, Nanjing, Jiangsu 211100 (CN)
(74) Representative: Synergy IP Group AG
(86) International application number: PCT/CN2023/109788
(87) International publication number: WO 2024/022474

(57) **Abstract**

Provided is a chimeric transcriptional activator for a methanol-inducible promoter, which at least comprises a first portion and a second portion, wherein: 1) the first portion comprises: i) a DNA binding domain; or ii) a DNA binding domain and a first transcription activation domain; and 2) the second portion comprises at least one second transcription activation domain. Further provided is a use of the chimeric transcriptional activator in enhancing foreign protein expression levels in a host cell.

## Description

### Cross Reference to Related Applications

The present application claims the priority of China patent application CN202210910746.0 filed on July 29, 2022, which is hereby incorporated herein by reference in its entirety.

### Technical Field

The present disclosure relates to a chimeric transcriptional activator, especially a chimeric transcriptional activator that can promote the transcription of *Pichia pastoris* AOX1 promoter. The present disclosure further relates to the use of the chimeric transcriptional activator in enhancing the expression of a protein of interest (such as hemoglobin) in *Pichia pastoris.*

### Background Art

Hemoglobins are a class of iron-containing metalloproteins with heme as a cofactor present in prokaryotic and eukaryotic cells and have numerous important functions in organisms such as transporting and storing oxygen, regulating intracellular pH value, and regulating physiological metabolism. In recent years, hemoglobin has been used in fields such as emergency medicine (as an acellular oxygen carrier), medical care (as an iron supplement), and food processing (as a food-grade coloring and flavoring agent for artificial meat). However, the acquisition of hemoglobin still necessarily relies on extraction from blood or plant tissues. The extraction method is not only time-consuming and inefficient, but also involves using chemical reagents that easily cause environmental pollution. Therefore, the synthesis of hemoglobin from different sources in microbial cell factories as a platform has become a hotspot of research in recent years.

*Pichia pastoris* is a commonly used high-efficiency protein expression system, which has the advantages of high expression levels, low glycosylation of proteins, high-density culture, etc., and is widely used in food and pharmaceutical fields. *Pichia pastoris* can grow using methanol as the sole carbon source, and alcohol oxidase AOX1 in its methanol utilization pathway accounts for 30% of the total proteins expressed by *Pichia pastoris.* Therefore, AOX1 promoter is a very strong methanol-inducible promoter and is often used to achieve efficient expression of heterologous proteins. It has been reported that there are three important transcriptional activators in the methanol metabolism pathway in *Pichia pastoris,* namely Mxr1, Mit1 and Prm1, respectively. These three transcription factors bind to different motifs of AOX1 promoter to coordinately regulate transcription activation.

Patent US 10273492 has reported that the expression of leghemoglobin can be improved by connecting AOX1 promoter to an Mxr1 coding sequence to express Mxr1 in *Pichia pastoris.* However, in order to reduce the cost, it is necessary to further improve the expression level.

### Summary of the Invention

In one aspect, provided herein is a chimeric transcriptional activator, comprising at least a first portion and a second portion, wherein:
1) the first portion comprises:
   i) a DNA binding domain; or
   ii) a DNA binding domain and a first transcription activation domain; and
2) the second portion comprises at least one second transcription activation domain.

In some embodiments, the C-terminal end of the first portion is connected to the N-terminal end of the second portion directly or indirectly via a linker molecule.

In some embodiments, the DNA binding domain and the second transcription activation domain are derived from different transcriptional activators.

In some embodiments, the DNA binding domain and the first transcription activation domain are derived from the same transcriptional activator.

In some embodiments, the DNA binding domain is derived from transcriptional activator Mxr1, Mit1, Prm1, Adr1, Trm1, Trm2, Mpp1, or fragments or mutants thereof, preferably from transcriptional activator Mxr1, Mit1, Prm1, or fragments or mutants thereof.

In some embodiments, the first transcription activation domain is derived from transcriptional activator Mxr1, Mit1, Prm1, Adr1, Trm1, Trm2, Mpp1, or fragments or mutants thereof, preferably from transcriptional activator Mxr1, Mit1, Prm1, or fragments or mutants thereof.

In some embodiments, the transcriptional activator Mxr1 comprises a sequence set forth in SEQ ID NO: 10; the transcriptional activator Mit1 comprises a sequence set forth in SEQ ID NO: 11; and the transcriptional activator Prm1 comprises a sequence set forth in SEQ ID NO: 12.

In some embodiments, the DNA binding domain comprises amino acids at positions 1-91 of the transcriptional activator Mxr1, amino acids at positions 1-104 of the transcriptional activator Mit1, or amino acids at positions 1-89 of the transcriptional activator Prm1.

In some embodiments, the DNA binding domain has at least 50%, 60%, 70%, 80%, 90%, 92%, 95%, 98%, or 99% sequence identity to amino acids at positions 1-91 of the transcriptional activator Mxr1, amino acids at positions 1-104 of the transcriptional activator Mit1, or amino acids at positions 1-89 of the transcriptional activator Prm1.

In some embodiments, the first transcription activation domain comprises amino acids at positions 92-1155 of the transcriptional activator Mxr1, amino acids at positions 92-400 of the transcriptional activator Mxr1, amino acids at positions 105-888 of the transcriptional activator Mit1, or amino acids at positions 90-989 of the transcriptional activator Prm1.

In some embodiments, the first transcription activation domain has at least 50%, 60%, 70%, 80%, 90%, 92%, 95%, 98%, or 99% sequence identity to amino acids at positions 92-1155 of the transcriptional activator Mxr1, amino acids at positions 92-400 of the transcriptional activator Mxr1, amino acids at positions 105-888 of the transcriptional activator Mit1, or amino acids at positions 90-989 of the transcriptional activator Prm1.

In some embodiments, the first portion comprises the transcriptional activator Mxr1, amino acids at positions 1-400 of the transcriptional activator Mxr1, amino acids at positions 1-91 of the transcriptional activator Mxr1, the transcriptional activator Mit1, amino acids at positions 1-104 of the transcriptional activator Mit1, the transcriptional activator Prm1, or amino acids at positions 1-89 of the transcriptional activator Prm1.

In some embodiments, the second transcription activation domain is derived from transcriptional activator Mxr1, Mit1, Prm1, Adr1, Trm1, Trm2, Mpp1, or fragments or mutants thereof, preferably from transcriptional activator Mxr1, Mit1, Prm1, or fragments or mutants thereof.

In some embodiments, the second transcription activation domain comprises amino acids at positions 92-1155 of the transcriptional activator Mxr1, amino acids at positions 92-400 of a truncated transcriptional activator mMxr1, amino acids at positions 105-888 of the transcriptional activator Mit1, or amino acids at positions 90-989 of the transcriptional activator Prm1.

In some embodiments, the second transcription activation domain has at least 50%, 60%, 70%, 80%, 90%, 92%, 95%, 98%, or 99% sequence identity to amino acids at positions 92-1155 of the transcriptional activator Mxr1, amino acids at positions 92-400 of a truncated transcriptional activator mMxr1, amino acids at positions 105-888 of the transcriptional activator Mit1, or amino acids at positions 90-989 of the transcriptional activator Prm1.

In some embodiments, the second portion comprises amino acids at positions 92-1155 of the transcriptional activator Mxr1, amino acids at positions 92-400 of a truncated transcriptional activator mMxr1, amino acids at positions 105-888 of the transcriptional activator Mit1, or amino acids at positions 90-989 of the transcriptional activator Prm1.

In some embodiments, the chimeric transcriptional activator comprises a sequence set forth in any one of SEQ ID NOs: 14-25.

In another aspect, provided herein is an isolated nucleic acid molecule, comprising a coding nucleic acid sequence for the above-mentioned chimeric transcriptional activator.

In another aspect, provided herein is an expression vector, comprising the above-mentioned nucleic acid molecule.

In some embodiments, in the expression vector, the coding nucleic acid sequence is operably connected to a methanol-inducible promoter.

In some embodiments, the methanol-inducible promoter includes AOX1, DAS1, DAS2, CAT1, and PMP20 promoters, or mutants thereof, preferably AOX1 promoter.

In another aspect, provided herein is a host cell, comprising the above-mentioned nucleic acid molecule or expression vector.

In another aspect, provided herein is a host cell, which expresses the above-mentioned chimeric transcriptional activator.

In some embodiments, the host cell further comprises a nucleic acid sequence encoding a heterologous polypeptide that is operably connected to a methanol-inducible promoter.

In some embodiments, the heterologous polypeptide is derived from the PF00042 globulin family.

In some embodiments, the heterologous polypeptide is hemoglobin, preferably soy leghemoglobin LegH or bovine myoglobin.

In some embodiments, the heterologous polypeptide comprises a sequence set forth in SEQ ID NO: 9 or SEQ ID NO: 26.

In some embodiments, the host cell further comprises a nucleic acid encoding at least one polypeptide involved in a heme biosynthesis pathway that is operably connected to the methanol-inducible promoter.

In some embodiments, the host cell is a yeast cell, preferably *Pichia pastoris,* more preferably *Pichia pastoris* X-33 strain.

In one aspect, provided herein is a method for preparing a protein of interest, comprising culturing the above-mentioned host cell and isolating the protein of interest.

In some embodiments, the host cell is *Pichia pastoris,* and the process of culturing the host cell comprises adding methanol to a culture solution.

In some embodiments, the protein of interest is a protein from the PF00042 globulin family.

In some embodiments, the protein of interest is hemoglobin, preferably soy leghemoglobin LegH or bovine myoglobin.

### Brief Description of the Drawings

Fig. 1 shows a schematic diagram of heme biosynthesis pathway.
Fig. 2 shows a schematic diagram of the construction of a plasmid for a soy leghemoglobin expression strain.
Fig. 3 shows an SDS-PAGE gel diagram of shake flask fermentation of each strain. M: Marker; 1-2: TF624; 3-5: TF625; 6: TF626; 7: TF627; 8: TF628; 9-10: TF629; 11-12: M202; 13: TF720; 14: TF721; 15-16: TF722; 17-18: TF723; 19-20: TF724; 21-22: TF725; 23: TF726; 24: TF727; 25: TF728; 26: TF729; 27-28: TF730; 29: TF731; and 30-31: L528.
Fig. 4 shows photos showing the color of a fermentation broth after shake flask fermentation of each strain.
Fig. 5 shows a schematic diagram of a pUC57-pAOX1-Mxr1-hph expression plasmid.

### Detailed Description of Embodiments

Unless otherwise stated, all technical and scientific terms used herein have the meanings commonly understood by those of ordinary skill in the art.

The term "or" refers to an individual element among the listed optional elements, unless otherwise explicitly stated in the context. The term "and/or" refers to any one of, any two of, any three or more of, or all of the listed optional elements.

As used herein, the term "about" means a value within a range of ± 10% of a given numerical value.

As used herein, the terms "comprising", "containing", "having" and similar expressions mean not excluding unlisted elements. These terms also include cases that consist only of the listed elements.

A "promoter" is a segment of DNA sequence where RNA polymerase recognizes, binds and initiates transcription. It contains a conserved sequence required by specific binding to RNA polymerase and transcription initiation, which is, in most cases, located upstream of a transcription start point of a structural gene, and the promoter itself is not transcribed. Examples of common promoters include but are not limited to promoters CMV, EF1A, CAG, CBh, SFFV, T7, etc. A promoter can comprise a core promoter and a regulatory sequence. The core promoter means the minimum sequence required for initiating transcription, such as a sequence called TATA box, which is common to most promoters for genes encoding proteins; and the regulatory sequence may comprise a DNA fragment that can be recognized and bound by a transcriptional activator or repressor, whereby the transcription initiation rate of the promoter may be affected after they bind the regulatory sequence portion.

An "inducible promoter", also known as "regulatory promoter", refers to a promoter that selectively expresses a coding sequence or a functional RNA in cells or tissues in response to the presence of endogenous or exogenous stimulation, such as by treatment with compounds (chemical inducers), or in response to the environment (changes in nutrients or carbon sources), hormones, developmental signals, etc. In other words, the inducible promoter initiates (or enhances) the transcription of the coding sequence operably connected thereto only under specific conditions. An example of inducible promoters is alcohol oxidase AOX1 of *Pichia pastoris,* which is activated only when methanol is present in the culture medium. Herein, the promoter that initiates the expression of related genes only in the presence of methanol is called a methanol-inducible promoter. Examples of methanol-inducible promoters include AOX1, DAS1, DAS2, CAT1, or PMP20 promoter, especially AOX1 promoter. A "constitutive promoter" corresponding to an inducible promoter expresses related genes in a relatively stable manner.

"Operably connected" means that a regulatory sequence is connected to its regulatory object in such a way that the regulatory sequence can act on its regulatory object. For example, a promoter being "operably connected" to a gene of interest means that the promoter can drive the transcription of the gene of interest from the exact start site; and a regulatory sequence being "operably connected" to a core promoter means that the regulatory sequence can control the transcription initiation rate of the core promoter.

A "linker molecule" refers to a segment of short peptide sequence, which can connect two or more protein domains and maintain a certain minimum distance or other spatial relationship between the protein domains and generally have no specific biological activity. Common linker molecules can be divided into three categories according to their structures: flexible linker molecules, rigid linker molecules, and cleavable linker molecules. Cleavable linker molecules cannot be used in the present disclosure. What is used in the present disclosure is a linker molecule that is usually not cleavable in a cell. Flexible linker molecules usually comprise non-polar amino acids such as glycine, or polar amino acids such as serine or threonine. Common flexible linker molecules include: (GGGGS)ₙ (SEQ ID NO: 27), (G)ₙ, (G)s, GSAGSAAGSGEF (SEQ ID NO: 28), KESGSVSSEQLAQFRSLD (SEQ ID NO: 29), or EGKSSGSGSESKST (SEQ ID NO: 30), where n represents the number of amino acid repeats in the preceding parentheses. Rigid linker molecules usually contain an α-helix structure or multiple prolines to form a relatively rigid structure. Common rigid linker molecules include: (EAAAK)ₙ (SEQ ID NO: 31) or (XP)ₙ, wherein n represents the number of amino acid repeats in the preceding parenthesis and X represents any amino acid, preferably alanine, lysine, or glutamic acid.

A "transcriptional activator recognition and binding site" refers to a segment of nucleotide sequence on a DNA molecule, which a transcription factor can recognize and bind to. The binding of a transcriptional activator thereto is helpful to form a transcription initiation complex with other proteins (such as RNA polymerase) and initiates the transcription process. The "transcriptional activator recognition and binding site" can be considered as a part of the regulatory sequence of a promoter.

A "domain" refers to a structural level between the secondary and tertiary structures of a protein or polypeptide, which is a distinguishable compact spherical structural region. For smaller proteins or protein subunits, a domain and a tertiary structure thereof often have the same meaning, that is to say, these proteins or subunits are single domains. For larger protein domains, two or more domains may be included. The domains themselves can be tightly assembled, but the relationship between domains is relatively loose. Domains are often connected by peptide chains of varying lengths, forming a so-called hinge region. The number of domains varies among different proteins, and the domains within the same protein molecule can be similar or very different from each other. The number of amino acid residues in common domains is between 100 and 400, and the smallest domain has only 40-50 amino acid residues, while large domains may have more than 400 amino acid residues.

A "transcriptional activator" herein refers to a protein (including a fusion protein) or polypeptide that can bind to part of the DNA sequence in a promoter and thus activate or enhance the transcriptional activity of the promoter. The transcriptional activator, alone or in combination with other proteins or polypeptides, can activate or enhance the transcriptional activity of the promoter. In some examples, a transcriptional activator may comprise at least two domains: a DNA binding domain, which is used to bind to a regulatory sequence in a promoter; and a transcription activation domain, which is used to activate or enhance the transcription initiation rate of the promoter. For example, transcriptional activators in yeast cells can include Mxr1, Mit1, Prm1, Adr1, Trm1, Trm2, Mpp1, etc. In some embodiments, the transcriptional activator may be operably connected to a methanol-inducible promoter, and the methanol-inducible promoter includes AOX1, DAS1, DAS2, CAT1, or PMP20 promoter, especially AOX1 promoter.

A "chimeric transcriptional activator" herein refers to a transcriptional activator that comprises a DNA binding domain and one or more transcription activation domains, wherein at least one domain and another domain do not naturally exist in the same transcriptional activator. For example, where transcriptional activator X comprises DNA binding domain A1 and transcription activation domain B1 and transcriptional activator Y comprises DNA binding domain A2 and transcription activation domain B2, a transcriptional activator comprising domains A1 and B2, a transcriptional activator comprising domains A1, B1 and B2, a transcriptional activator comprising domains A2 and B1, and a transcriptional activator comprising domains A2, B1 and B2 can all be considered as chimeric transcriptional activators. In some embodiments herein, the DNA binding domain and the transcription activation domain comprised in the chimeric transcriptional activator are derived from transcriptional activators of *Pichia pastoris.* It can be envisioned that transcription factors can exist widely in different host cells. Therefore, these DNA binding domains and transcription activation domains may be obtained or derived from other prokaryotes or eukaryotes, instead of using natural sequences of specific species or genera. Preferably, these transcriptional activator sequences can be obtained or derived from fungal host cells, more preferably obtained or derived from yeast host cells such as *Pichia pastoris, Hansenula polymorpha, Trichoderma reesei, Aspergillus niger, Saccharomyces cerevisiae, Kluyveromyces lactis, Yarrowia lipolytica, Pichia methanolica, Candida boidinii, Komagataella* spp. *Schizosaccharomyces pombe,* etc. In some embodiments, the DNA binding domain and the transcription activation domain of the chimeric transcriptional activator are derived from transcriptional activator Mxr1, Mit1, Prm1, Adr1, Trm1, Trm2, or Mpp1. Preferably, the DNA binding domain of the chimeric transcriptional activator comprises amino acids at positions 1-91 of the transcriptional activator Mxr1, amino acids at positions 1-104 of the transcriptional activator Mit1, or amino acids at positions 1-89 of the transcriptional activator Prm1, or functional variants thereof. Preferably, the transcription activation domain comprises amino acids at positions 92-1155 of the transcriptional activator Mxr1, amino acids at positions 92-400 of the transcriptional activator Mxr1, amino acids at positions 105-888 of the transcriptional activator Mit1, or amino acids at positions 90-989 of the transcriptional activator Prm1, or functional variants thereof.

"Functional variant" and "mutant" are used interchangeably herein and refer to a protein or nucleotide variant obtained by incorporating a few modifications (such as amino acid or nucleotide deletion, addition or substitution) on the basis of the original sequence (such as a natural sequence) or a sequence provided herein but still retaining all or at least some of the functions of the parent sequence. For example, the functional variant can have at least about 50%, 60%, 70%, 80%, 90%, 92%, 95%, 98%, or 99% sequence identity to the parent sequence and can retain about 50%, 60%, 70%, 80%, 90%, or 100% of some activity of the parent sequence or even have an activity higher than that of the parent sequence. The "functional variant" or "mutant" may be artificially produced (such as by genetic manipulation) or naturally produced in nature (e.g., protein polymorphisms in a population or homologous proteins existing in different species). In addition, the "functional variant" or "mutant" may also be a functional fragment of the original sequence, such as the smallest fragment that retains the function of concern, e.g., a DNA binding domain with DNA binding ability. Herein, when referring to a specific amino acid or nucleotide sequence, it should be recognized that it may cover functional variants or mutants of such amino acids or nucleotide sequences, unless otherwise stated or implied in the context.

When referring to an amino acid or nucleotide sequence, the term "sequence identity" refers to the degree of identity between two amino acid or nucleotide sequences (such as a query sequence and a reference sequence), generally expressed as a percentage. Generally, before calculating the percentage of identity between two amino acid or nucleotide sequences, sequence alignment is performed first, and gaps (if any) are introduced. If amino acid residues or bases are the same at a certain alignment position in two sequences, the two sequences are believed to be consistent or matched at this position; and if the amino acid residues or bases are different in the two sequences, they are believed to be inconsistent or mismatched at this position. In some algorithms, the number of matched positions is divided by the total number of positions in the alignment window to obtain sequence identity. In other algorithms, the number of gaps and/or the gap length is also taken into account. Commonly used sequence comparison algorithms or softwares include DANMAN, CLUSTALW, MAFFT, BLAST, MUSCLE, etc. For the purpose of the present disclosure, the publicly available alignment software BLAST (available at https://www.ncbi.nlm.nih.gov/) can be used to obtain the optimal sequence alignment by using default settings and calculate the sequence identity between two amino acid or nucleotide sequences.

"Nucleic acid molecule", "nucleic acid" and "polynucleotide" are used interchangeably herein and refer to a polymer of nucleotides. Such polymers of nucleotides may contain natural and/or unnatural nucleotides and include, but are not limited to, DNA, RNA and PNA. A "nucleic acid sequence" refers to a linear sequence of nucleotides comprised in a nucleic acid molecule or polynucleotide. For DNA molecules, due to double-strand complementation, when the sequence of one strand is mentioned herein, those skilled in the art should recognize that its complementary strand or a double-stranded DNA molecule comprising its complementary strand has also been mentioned.

Herein, an "isolated nucleic acid molecule" refers to an extracted or purified nucleic acid molecule and also includes artificially synthesized nucleic acid molecules. For example, it can be produced or synthesized by the following methods: (i) amplification *in vitro,* for example, production by polymerase chain reaction (PCR) amplification, (ii) cloning and recombination, (iii) purification, for example, fractionation by restriction enzyme digestion and gel electrophoresis, or (iv) synthesis, for example, by chemical synthesis. In certain embodiments, the isolated nucleic acid molecule is a nucleic acid molecule prepared by recombinant DNA technology.

A "vector" refers to a nucleic acid molecule that can be engineered to contain a polynucleotide of interest (e.g., a coding sequence for the polypeptide of interest) or a nucleic acid molecule (e.g., a nucleic acid, a plasmid, or a virus) that can be replicated in a host cell. The vector may comprise one or more of the following components: an origin of replication, one or more regulatory sequences that regulate the expression of the polynucleotide of interest, an enhancer, a promoter and/or one or more selectable marker genes (such as antibiotic resistance genes and genes that can be used in colorimetric analysis, such as the β-galactose gene). The term "expression vector" refers to a vector for expressing a gene of interest in a host cell.

A "host cell" refers to a cell that can be or has been a recipient of a vector or an isolated polynucleotide. The host cell can be a prokaryotic cell or a eukaryotic cell. Exemplary eukaryotic cells include mammalian cells, such as primate or non-primate cells; fungal cells, such as yeast; plant cells; and insect cells. Host cells include the offspring of a single host cell, and the offspring may not be exactly the same as the original parent cell (in terms of morphology or genomic DNA complementarity) due to natural, accidental or intentional mutation. Host cells also include cells transfected with nucleic acid molecules or expression vectors provided herein *in vivo.*

A "gene of interest" refers to a polynucleotide sequence encoding an RNA or protein product, which polynucleotide sequence can be introduced into a cell or an individual according to a desired purpose and can be expressed under suitable conditions. The gene of interest may encode a product of interest, such as a therapeutic or diagnostic product of interest. The protein product encoded by the gene of interest is called a protein of interest. The protein of interest is usually a heterologous polypeptide or foreign polypeptide, that is, the heterologous polypeptide or foreign polypeptide is usually not expressed in the host cell; instead, the host cell is artificially engineered such that the heterologous polypeptide or foreign polypeptide can be expressed therein.

A "heterologous polypeptide" refers to a polypeptide encoded by any nucleic acid sequence that is not natural relative to the genome or biological genome. In the present disclosure, the heterologous polypeptide may be derived from a member of the PF00042 (Pfam database protein family ID number) globulin family. In some embodiments of the present disclosure, a polypeptide encoding soy *(Glycine max)* leghemoglobin LegH or a polypeptide sequence encoding bovine *(Bos taurus)* myoglobin may exist in a host cell. The heterologous polypeptide can be expressed in a methylotrophic yeast strain and can bind to a methanol-inducible or constitutive promoter or an element thereof to further enhance the expression of the heterologous nucleic acid.

A "foreign polypeptide" or "foreign protein" refers to a polypeptide encoded by any nucleic acid sequence introduced into the genome of a cell from an external source, wherein the external source may be the same or a different organism or a polypeptide produced synthetically. For example, the foreign polypeptide may be a polypeptide derived from a microorganism (for example, a genus or a species of methylotrophic yeast), which is introduced into a methylotrophic yeast of a different genus or species. However, the foreign polypeptide may also be a polypeptide from a methylotrophic yeast, which is recombinantly introduced into the methylotrophic yeast as an extra copy despite the presence of a corresponding natural nucleic acid sequence. In the present disclosure, the coding nucleic acid sequences for eight enzymes involved in a heme cofactor biosynthesis pathway are endogenous genes of *Pichia pastoris,* including 5-aminolevulinate synthase, porphobilinogen synthase, porphobilinogen deaminase, uroporphyrinogen III synthase, uroporphyrinogen decarboxylase, coproporphyrinogen oxidase, protoporphyrinogen oxidase, and ferrochelatase. These eight enzyme-encoding genes exogenously expressed through recombination and integrated into *Pichia pastoris* to further enhance the expression of nucleic acids are considered to be exogenous.

There are few reports on chimeric transcription factors of *Pichia pastoris.* In the present disclosure, a variety of artificial chimeric transcription factors are designed. Testing whether various chimeric transcription factors have activity and screening out artificial transcription factors that are most beneficial to protein expression is of great significance for further improving the expression of heterologous proteins.

In the present disclosure, several groups of chimeric transcriptional activators are designed, which are transformed into hemoglobin expression strains containing the heme synthesis pathway. Compared with pAOX1-Mxr1, screened chimeric transcriptional activators Mxr1D-Mit1AD, Mxr1D-Prm1AD, etc., can further improve the expression of hemoglobin.

The present disclosure is based in part on the surprising discovery that the expression (or overexpression) of at least one chimeric transcription factor described herein in a host cell (such as *Pichia pastoris)* is found to increase the yield of a recombinant protein of interest (a foreign protein, such as soy leghemoglobin or bovine myoglobin). Therefore, in one aspect, provided herein is a novel chimeric transcriptional activator; and in another aspect, provided herein is a method for increasing the yield of a foreign protein in a host cell, comprising expressing one or more of these chimeric transcriptional activators in the host cell. Compared with host cells that do not express polynucleotides encoding these chimeric transcriptional activators, host cells that express the polynucleotides encoding these chimeric transcriptional activators increase the yield of foreign proteins. In addition, the inventors have also found that overexpression of heme in a host cell helps foreign proteins such as soy leghemoglobin or bovine myoglobin to better bind heme in nascent proteins.

### Example 1. Introduction of heme pathway into Pichia pastoris' genome

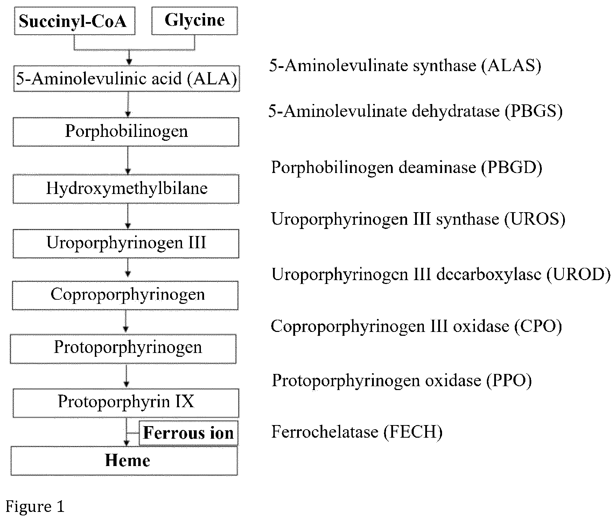

Heme consists of a porphyrin ring and an iron ion. Heme, as an important component of hemoglobin, forms a complete hemoglobin through covalent linkage with globin. In order to obtain high-yield hemoglobin, sufficient heme is necessary in cells for hemoglobin synthesis. Heme biosynthesis pathway in S. *cerevisiae* has been reported (as shown in Fig. 1), including eight enzymes, namely, 5-aminolevulinate synthase (ALA synthase, ALAS), porphobilinogen synthase (PBGS), porphobilinogen deaminase (PBGD), uroporphyrinogen III synthase (UROS), uroporphyrinogen III decarboxylase (UROD), coproporphyrinogen III oxidase (CPO), protoporphyrinogen oxidase (PPO), and ferrochelatase (FECH). ALA synthase synthesizes the precursor substances L-glycine and succinyl-CoA into the key precursor 5-aminolevulinate (ALA), which is then synthesized into heme under the catalysis action of the above-mentioned various enzymes. The eight enzyme-encoding genes in the heme biosynthesis pathway in the genome of *Pichia pastoris* were searched in NCBI. The sequences are shown in SEQ ID NOs: 1-8.

The eight enzyme-encoding genes in the heme biosynthesis pathway were integrated into the genome of *Pichia pastoris.* Using genome of *Pichia pastoris* X-33 as a template, the eight related enzyme-encoding genes in the heme biosynthesis pathway were amplified respectively, and the above-mentioned genes were respectively connected to the AOX1 promoter and the FDHI terminator or the actin terminator by means of digestion and linkage to form expression cassettes. The above-mentioned eight genes were divided into four groups, namely, ALAS and PBGD, PPO and FECH, UROS and PBGS, and UROD and CPO. Every two expression cassettes were connected in series, then respectively connected to upstream and downstream homologous arms, the positive selection marker Zeocin resistance gene, and the negative selection marker mazF gene, and assembled onto pUC57 plasmids to obtain heme synthesis pathway expression plasmids. Through homologous recombination in yeasts, the eight genes in the heme synthesis pathway were integrated into the genome of the *Pichia pastoris* X-33 strain in four steps to obtain *Pichia pastoris* strain HEM201 with enhanced heme biosynthesis pathway.

### Example 2. Construction of Pichia pastoris soy leghemoglobin expression strain

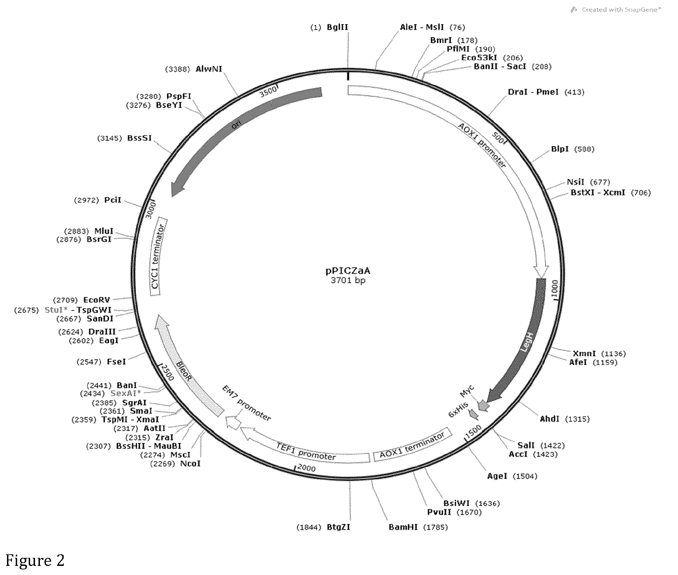

Soy leghemoglobin gene LegH (SEQ ID NO: 9) was ligated into pPIZa expression vector (as shown in Fig. 2), linearized by SacI, and then transformed into the *Pichia pastoris* strain HEM201 in Example 1 to obtain soy leghemoglobin expression strain L528.

The expression of hemoglobin was verified by shake flask fermentation (50 mL/250 mL) of the *Pichia pastoris* recombinant strain L528.

The constructed strain was streaked and cultured for 2-3 days until a single colony had grown. The single colony was picked, inoculated into YPD medium (50 mL/250 mL), shaken at 30°C and 100-600 rpm until OD600 = 2-6, the culture was then centrifuged at 4°C and 1500-3000 g for 5-10 minutes, and the cells were washed twice with normal saline to remove glucose from the culture medium. After centrifugation, all the pellets were suspended in 50 mL of BMMY culture medium and fermented at 30°C and 250 rpm (heme with a final concentration of 20 mg/L was added to the culture medium at the beginning of fermentation). 1% methanol + 0.1 mM ammonium ferric citrate (final concentration) was added to the fermentation broth every 24 hours for continuous induction, and the fermentation broth was sampled at 120 h for analysis.

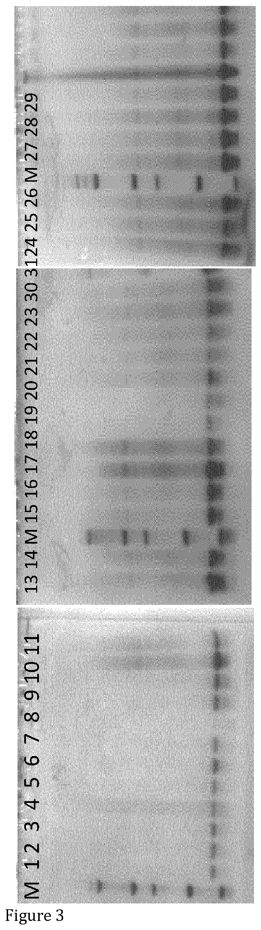

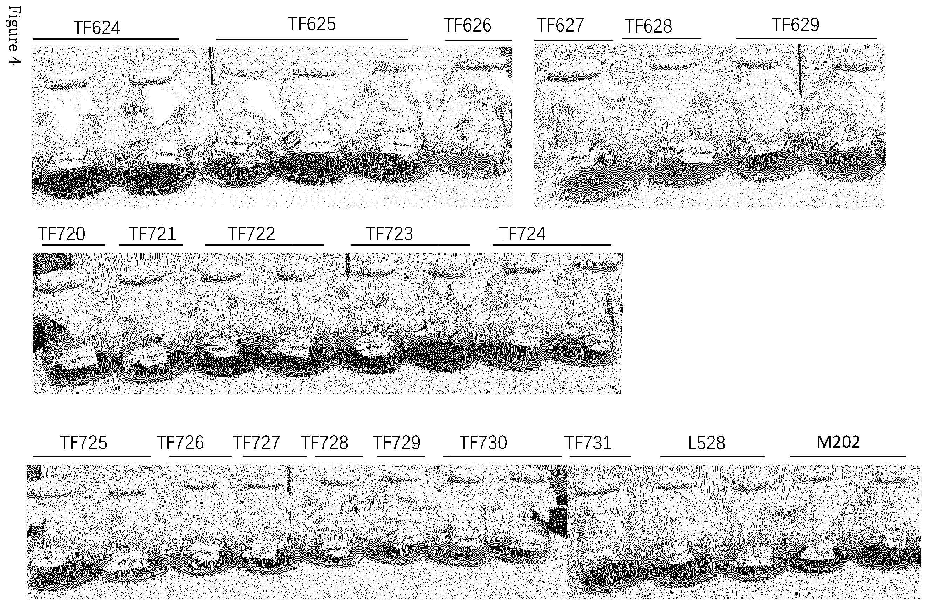

The results of shake flask fermentation showed that soy leghemoglobin was expressed, with obvious bands in the SDS-PAGE gel (Fig. 3), and the fermentation broth was pink (Fig. 4).

### Example 3. Introduction of known yeast transcription factors into Pichia pastoris soy leghemoglobin expression strains

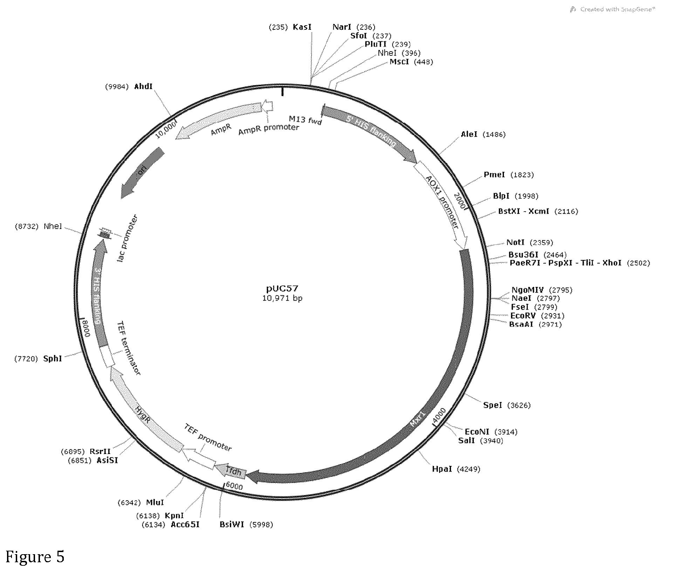

An expression vector pMxr1 for transcriptional activator Mxr1 (SEQ ID NO: 10) for the methanol metabolism pathway in *Pichia pastoris* was constructed. Using the genome of *Pichia pastoris* X-33 as a template, the Mxr1 gene was amplified, and through overlap PCR, the Mxr1 gene was ligated between AOX1 promoter and FDH terminator to form an Mxr1 expression cassette. The Mxr1 expression cassette, upstream and downstream homologous arms at positions upstream of HIS gene, and hygromycin hph gene were ligated into pUC57 plasmid to obtain Mxr1 expression vector pUC57-pAOX1-Mxr1-hph (as shown in Fig. 5).

The plasmid pUC57-pAOX1-Mxr1-hph was linearized with restriction endonuclease NheI and transformed into soy leghemoglobin expression strain L528. The Mxr1 gene under the control of AOX1 promoter was inserted upstream of the position of HIS gene in the *Pichia pastoris'* genome to obtain soy leghemoglobin expression strain M202 with enhanced Mxr1 gene.

In the results of shake flask fermentation, the SDS-PAGE gel diagram showed that the expression level of soy leghemoglobin in the M202 strain was increased than that in the L528 strain (as shown in Fig. 3), and the fermentation broth was red (as shown in Fig. 4).

In addition to the transcription factor Mxr1, the other transcriptional activators for methanol metabolism pathway may also be beneficial for improving the expression of soy leghemoglobin and turning the fermentation broth red. Two other important transcription factors Mit1 and Prm1 for the methanol metabolism pathway of *Pichia pastoris* and transcription factor Mpp1 of *H. polymorpha* were respectively selected for expression (SEQ ID NOs: 11-13). The strain construction method was the same as that for the strain M202. The expression of the transcription factors was regulated by AOX1 promoter and GAP promoter, respectively, and expression cassettes were constructed. The expression cassettes of different transcription factors, upstream and downstream homologous arms at positions upstream of HIS gene, and hygromycin hph gene were ligated into pUC57 plasmid to obtain different transcription factor expression vectors pTF624-pTF629 (as shown in Table 1).

The vectors were linearized with restriction endonuclease NheI and then transformed into soy leghemoglobin expression strain L528. Different transcription factor genes under the control of AOX1 promoter were inserted upstream of the position of HIS gene in the *Pichia pastoris'* genome to obtain soy leghemoglobin expression strains TF624-TF629 with enhanced transcription factor genes.

According to the shake flask fermentation method in Example 2, the expression of hemoglobin was verified. In the results of shake flask fermentation, the SDS-PAGE gel diagram showed that the expression levels in the soy leghemoglobin strains TF624-TF629 were not as high as that in the M202 strain (as shown in Fig. 3), and except for the fermentation broths of TF624 and TF625 which showed red colors close to that of M202, the fermentation broths of the other strains showed light red and light yellow colors (as shown in Fig. 4).

### Example 4. Introduction of chimeric yeast transcription factors into Pichia pastoris soy leghemoglobin expression strain

According to literature reports and NCBI BLAST analysis, the amino acid sequences of DNA binding domains (DBDs) and transcription activation domains (TADs) of three important transcription factors (Mxr1, Mit1, and Prm1) for *Pichia pastoris* were obtained and recombined. It has been reported that amino acids at positions 1-400 of Mxr1 (mMxr1) can retain the complete functions of Mxr1. DNA binding domain Mxr1D of Mxr1 comprises amino acids at positions 1-91, and transcription activation domain mMxr1AD comprises amino acids at positions 92-400; DNA binding domain Mit1D of Mit1 comprises amino acids at positions 1-104, and transcription activation domain Mit1AD comprises amino acids at positions 105-888; and DNA binding domain Prm1D of Prm1 comprises amino acids at positions 1-89, and transcription activation domain Prm1AD comprises amino acids at positions 90-989.

Different DNA binding domains and transcription activation domains were recombined to obtain various novel chimeric transcription factor sequences. The chimeric transcription factors were constructed as follows:
truncated mMxr1 and transcription activation domain mMxr1AD of truncated Mxr1 were connected via a GGGGS linker to further improve the transcriptional activation effect, whereby chimeric transcription factor mMxr1-mMxr1AD (SEQ ID NO: 14) was obtained;
Mxr1 and transcription activation domain Prm1AD of Prm1 were connected via a GGGGS linker to further improve the transcriptional activation effect, thereby chimeric transcription factor Mxr1-Prm1AD (SEQ ID NO: 15) was obtained;
DNA binding domain Mxr1D of Mxr1 and transcription activation domain Mit1AD of Mit1 were connected via a GGGGS linker to obtain the chimeric transcription factor Mxr1D-Mit1AD (SEQ ID NO: 16);
DNA binding domain Mxr1D of Mxr1 and transcription activation domain Prm1AD of Prm1 were connected via a GGGGS linker to obtain the chimeric transcription factor Mxr1D-Prm1AD (SEQ ID NO: 17);
Mit1 and transcription activation domain mMxr1AD of truncated mMxr1 were connected via a GGGGS linker to further improve the transcriptional activation effect, thereby chimeric transcription factor Mit1-mMxr1AD (SEQ ID NO: 18) was obtained;
DNA binding domain Mit1D of Mit1 and transcription activation domain mMxr1AD of truncated mMxr1 were connected via a GGGGS linker to further improve the transcriptional activation effect, thereby chimeric transcription factor Mit1D-mMxr1AD (SEQ ID NO: 19) was obtained;
DNA binding domain Mit1D of Mit1 and transcription activation domain Mxr1AD of Mxr1 were connected via a GGGGS linker to obtain the chimeric transcription factor Mit1D-Mxr1AD (SEQ ID NO: 20);
DNA binding domain Mit1D of Mit1 and transcription activation domain Prm1AD of Prm1 were connected via a GGGGS linker to obtain the chimeric transcription factor Mit1D-Prm1AD (SEQ ID NO: 21);
Prm1 and transcription activation domain mMxr1AD of truncated mMxr1 were connected via a GGGGS linker to further improve the transcriptional activation effect, thereby chimeric transcription factor Prm1-mMxr1AD (SEQ ID NO: 22) was obtained;
DNA binding domain Prm1D of Prm1 and transcription activation domain mMxr1AD of truncated mMxr1 were connected via a GGGGS linker to further improve the transcriptional activation effect, thereby chimeric transcription factor Prm1D-mMxr1AD (SEQ ID NO: 23) was obtained;
DNA binding domain Prm1D of Prm1 and transcription activation domain Mxr1AD of Mxr1 were connected via a GGGGS linker to obtain the chimeric transcription factor Prm1D-Mxr1AD (SEQ ID NO: 24); and
DNA binding domain Prm1D of Prm1 and transcription activation domain Mit1AD of Mit1 were connected via a GGGGS linker to obtain the chimeric transcription factor Prm1D-Mit1AD (SEQ ID NO: 25).

The above-mentioned transcription factors were substituted for the Mxr1 gene in pUC57-pAOX1-Mxr1-hph to obtain vector plasmids pTF720-pTF731 which contained different chimeric transcription factors (as shown in Table 1). The above-mentioned vector plasmids were linearized with restriction endonuclease NheI and then transformed into soy leghemoglobin expression strain L528. Different chimeric transcription factor genes under the control of the AOX1 promoter were inserted upstream of the position of the HIS gene in the *Pichia pastoris'* genome to obtain soy leghemoglobin expression strains TF720-TF731 correspondingly containing chimeric transcription factors.

According to the shake flask fermentation method in Example 2, the expression of hemoglobin was verified. Compared with the M202 strain, the expression levels in the strains TF722-TF723 were increased, and the colors of the fermentation broths were redder than that of the M202 strain. The expression levels in the TF730-TF731 strains were comparable to that in the M202 strain, but the colors of the fermentation broths were not as good as that of M202. None of the strains containing the other chimeric transcription factors had expression levels and fermentation broth colors as good as M202 (as shown in Figs. 3 and 4). The experimental results indicated that the expression effects of the chimeric transcription factors Mxr1D-Mit1AD and Mxr1D-Prm1AD were superior to the expression of Mxr1 and even superior to the expression effect of the other chimeric transcription factors.

**Table 1. Plasmids containing different chimeric transcription factors**

| Plasmid No. | Name of plasmid | Name of chimeric transcription factor |
|---|---|---|
| pTF624 | pUC57-pAOX1-Mit1-hph | Mit1 |
| pTF625 | pUC57-pGAP-Mit1-hph | Mit1 |
| pTF626 | pUC57-pAOX1-Prm1-hph | Prm1 |
| pTF627 | pUC57-pGAP-Prm1-hph | Prm1 |
| pTF628 | pUC57-pAOX1-Mpp1-hph | Mpp1 |
| pTF629 | pUC57-pGAP-Mpp1-hph | Mpp1 |
| pTF720 | pUC57-pAOX1-mMxr1-mMxr1AD-hph | mMxr1-mMxr1AD |
| pTF721 | pUC57-pAOX1-Mxr1-Prm1AD-hph | Mxr1-Prm1AD |
| pTF722 | pUC57-pAOX1-Mxr1D-Mit1AD-hph | Mxr1D-Mit1AD |
| pTF723 | pUC57-pAOX1-Mxr1D-Prm1AD-hph | Mxr1D-Prm1AD |
| pTF724 | pUC57-pAOX1-Mit1-mMxr1AD-hph | Mit1-mMxr1AD |
| pTF725 | pUC57-pAOX1-Mit1D-mMxr1AD-hph | Mit1D-mMxr1AD |
| pTF726 | pUC57-pAOX1-Mit1D-Mxr1AD-hph | Mit1D-Mxr1AD |
| pTF727 | pUC57-pAOX1-Mit1D-Prm1AD-hph | Mit1D-Prm1AD |
| pTF728 | pUC57-pAOX1-Prm1-mMxr1AD-hph | Prm1-mMxr1AD |
| pTF729 | pUC57-pAOX1-Prm1D-mMxr1AD-hph | Prm1D-mMxr1AD |
| pTF730 | pUC57-pAOX1-Prm1D-Mxr1AD-hph | Prm1D-Mxr1AD |
| pTF731 | pUC57-pAOX1-Prm1D-Mit1AD-hph | Prm1D-Mit1AD |

The intensities of the protein expression level and the fermentation broth color in the shake flask fermentation results of each strain were as shown in Table 2. The more "+" signs, the higher the protein expression level and the redder the fermentation broth color.

**Table 2. Shake flask fermentation results**

| Strain No. | Protein expression level | Fermentation broth color |
|---|---|---|
| L528 | + | + |
| M202 | +++ | +++ |
| TF624 | + | +++ |
| TF625 | + | +++ |
| TF626 | + | + |
| TF627 | + | + |
| TF628 | + | + |
| TF629 | + | + |
| TF720 | ++ | + |
| TF721 | ++ | + |
| TF722 | +++ | ++++ |
| TF723 | +++ | ++++ |
| TF724 | + | + |
| TF725 | + | + |
| TF726 | ++ | + |
| TF727 | + | + |
| TF728 | + | + |
| TF729 | + | + |
| TF730 | +++ | + |
| TF731 | +++ | + |

Some amino acid sequences mentioned herein are as follows:
>1 ALAS 
MEFVARQSMNACPFVRSTSTHHLKKLAANSSLAATASHCPVVGPALQQQRYYSQPSKPAQAQTSDIAT
GIKKDVSPIRMDSNETAFDYNGMYESDLANKRKDNSYRYFNNINRLAKEFPKAHRQTEDDKVTVWCS
NDYLGMGRHPEIIKTMKATMDKYGSGAGGTRNIAGHNHAAINLESELACLNKKEAALVFSSCFIANDAI
ISLLGQKIKNLVIFSDQSNHASMILGVRNSKAKKHIFKHNNLKDLESQLAQYPKSTPKLIAFESVYSMCGS
VAPIEKICDLAKRYGALTFLDEVHAVGMYGPHGQGVAEHLDFDLHLQSGIASPSVVDKRTILDRVDMIT
GTCGKSFGTVGGYVAGSANLIDWLRSYAPGFIFTTTLPPAIMAGTATSVRIVRADIEARIKQQLNTRYVK
DSFENLGIPVIPNPSHIVPVLVGNAADAKKASDMLMNKHRIYVQAINYPTVPVGEERLRITPTPGHGKEI
CDQLISAVDDVFTELNLPRINKWQSQGGHCGVGDANYVPEPNLWTQDQLSLTNQDLHSNVHNPVIEQI
ETSSGVRL* (SEQ ID NO: 1)
>2 PBGS 
MVHKAEYLDDHPTQISSILSGGYNHPLLREWQHERQLNKNMFIFPLFVTDRPDEEELIPSLPNIKRFGV
NKLIPYVGGLVSKGLRAVILFGVPLKPGVKDEEGTAADDPEGPVIQAIKHLRKNFPDLYIITDVCLCEYTS
HGHCGILYEDGTINRELSVRRIAAVAVKYAQAGANSVAPSDMTDGRIRDIKEGLLSAGLAHKTFVMSYA
AKFSGNLYGPFRDAAGSCPSQGDRKCYQLPSGGKGLAHRALIRDMNEGTDGIIVKPSTFYLDIVADAYQL
CKDYPICCYQVSGEYAMLHAAAEKNIVDLKSIAFEAHQGFLRAGARLIISYFTPEFLEWLSE* (SEQ ID
NO: 2)
>3 PBGD 
MNQIEQSGPIDCSSLKLGSRKSALAIIQAEIVRQLILKEYPELETKLVSVSTLGDQVQNKALFTFGGKSLW
TKELEMLLLESVGGFDQIDMIVHSLKDMPTHLPDEFELGCIIEREDPRDALVVQDGLSYKSLADLPEGA
VVGTSSVRRSAQLLKNFPHLKFKSVRGNLQTRLRKLDDPDSEYCCLLLAAAGLIRTGLQHRISMYLNDD
VMYHSVGQGALGVEIRKGDQFMKNICEKIGHRTTTLRCLAERALLRYLEGGCSVPIGVSTIYSEDTKELT
MNSLVVSCNGRDSVTESMTEVVTTEEQAEDFGERLAQKLIDQGAKRILDEINFNKIKEIKEEGLH* (SEQ
ID NO: 3)
>4 UROS 
MPKAILLKNKTTPKDPYLENFVSSGYSTDFVPLLDHIHMEKSEIIAFLKTDYFLHKTLAFIITSQRAVEML
NECMQILRRTDPEITQIIYSKPVYTVGPATYRILADAGFVDLRGGDKAGNGSILAQIILNDDIYTGIEDSDK
HITFFTGETRRDIIPKCLLSNNFQLYEKIVYKTLPRDDIVTRFKSAVDSIDQSQRSSSWVVFFSPQGTEDI
VTYLQHTKDQFNIASIGPTTEKYLLSKNLKPKVVAPKPEPISLLLSIQKVH* (SEQ ID NO: 4)
>5 UROD 
MSRFPELKNDLILRAARGEKVERPPIWIMRQAGRYLPEYHEVKGGRDFFETCRDAEIASEITIQPITHFD
GLIDAAIIFSDILVIPQAMGMEVKMVDKVGPQFPNPLRKPSDLDHLKKDVDVLKELDWAFKAISLTRKK
LNGRVPLLGFCGAPWTLLVYMTEGGGTKMFRFAKEWIYKFTKESHQLLQQITDVAVEFLAQQVVAGA
QMLQVFESWGGELGPDEFDEFSLPYLRQISSKLPLRLKELGITENVPITVFAKGSWYALEQLCDSGYDVV
SLDWLFRPSDAVQIANGRIALQGNLDPGTMYGSKETISKKVDKMIKGFGGGKQNYIINFGHGTHPFMD
PEQIRWFLQECHRIGSQ* (SEQ ID NO: 5)
>6 CPO 
MAIDSDINLSSPNDSIRQRMFELIQRKQLEIVAALEAIEGNDTKFRSDSWERGAEGGGGRSMLIQDGRVF
EKAGVNISKVHGVLPPQAVSQMRNDHSKLDLPAGTSLKFFACGLSLVIHPHNPHAPTTHLNYRYFETW
DETGKPHTWWFGGGADLTPSYLYPEDAKQFHQAHKDALDKHDVSLYPRFKKWCDEYFLIKHRNETR
 
GIGGIFFDDFDEFDAERSLKLVEDCFNAFLESYPAITRKRMDTPSTDAEKNWQQIRRGRYVEFNLVLDR
GTQFGLRTPGSRVESILMSLPRTAGWVYDHHPEPGSREEELLQVLQNPIEWV* (SEQ ID NO: 6)
>7 PPO 
MLKSLAPNSSIAVLGSGISGLTFSFFLNRLRPDVKIHIFEKSKQVGGWIRSEEHETFHFEKGPRTLRGTNT
GTLMLLDLLTKIGANDKVLGLHKDSLANKKYLLSPFSDVHGNNAKLLQVPQDFSSFVKFMFDPLSKDLI
LGLLKEPWQPKLKYSDESVDHFFNRRFATKLSENIVSAIVHGIYAGDVKKLSVKAIFPRLPEMEQESGSII
RYMIAQYRTKKNVKQKVDPFLADYEKLIGTSLSFKNISLFLKNFPMLSFQGGLQKLPISLKNHLSQIENIK
FHFDSKIKNIALESGKVALTDHDQVYLVDHVRSTINTNELAKIISPVVPSSTKKKSVFKSKANGPGLVKC
LSWLHYTNILMCNIYIPKHVSKSITGFGYLVPRSMSSQASKLLGVIFDSDIETAMTPNFTEANITAINSNS
ASPKQLQKFSDQFVNNDLPKYTKLTLMLGGHYLKSEADMPGSAESKHAVKAILSNHLNIDLDEFASLP
DFKMEITKIPNCIPQYEVGYLDLKRKVQNAASKEFNDQISFGGMAFGDGVGIPDCVQNAFKDSATLSGI*
(SEQ ID NO: 7)
>8 FECH 
MLNRRFQSTVSSSLNKGTGIVFMNMGGPSTVKETYDFLFRLFSDGDLIPFGRFQNILARFIASRRTPKIES
YYKAIGGGSPIRKWSEYQSSKLCEKLDIISPQSAPHKPYVAFRYANPLTEDTLQKMKNDGITKAIAFSQY
PQFSYSTTGSSINELYRQSKILDPDQSIKWTVIDRWPDHPALVKTFAAHIKDTLNRFKTENGLTDTKDV
VLQFSAHSLPMDIVNKGDSYPAEVAASVFAIMKELNFSNPYKLTWQSQVGPKPWLGAQTEKITKQLAS
SDVPGVVLVPIAFTSDHIETLHELDIELIQELPNPSKVKRVESLNGDQTFIDSLAELVKSHIDSKVVFSNQ
LPLDSMLGVVSDNSLTDPKEFFRAH* (SEQ ID NO: 8)
>9 LegH 
MGAFTEKQEALVSSSFEAFKANIPQYSVVFYTSILEKAPAAKDLFSFLSNGVDPSNPKLTGHAEKLFGLV
RDSAGQLKANGTVVADAALGSIHAQKAITDPQFVVVKEALLKTIKEAVGDKWSDELSSAWEVAYDELA
AAIKKAF* (SEQ ID NO: 9)
>10 Mxr1 
MSNLPPTFGSTRQSPEDQSPPVPKELSFNGTTPSGKLRLFVCQTCTRAFARQEHLKRHERSHTKEKPFS
CGICSRKFSRRDLLLRHAQKLHSNCSDAAITRLRRKATRRSSNAAGSISGSTPVTTPNTMGTPEDGEKR
KVQKLAGRRDSNEQKLQLQQQHLQQQPQLQYQQSLKQHENQVQQPDQDPLISPRMQLFNDSNHHVN
NLFDLGLRRASFSAVSGNNYAHYVNNFQQDASSTNPNQDSNNAEFENIEFSTPQMMPVEDAETWMN
NMGPIPNFSLDVNRNIGDSFTDIQHKNSEPIISEPPKDTAPNDKKLNGYSFYEAPIKPLESLFSVRNTKR
 
NKYKTNDDSPDTVDNNSAPAANTIQELESSLNASKNFCLPTGYSFYGNLDQQTFSNTLSCTSSNATISPI
LLDNSINNNSTSDVRPEFRTQSVTSEMSQAPPPPQKNNSKYSTEVLFTSNMRSFIHYALSKYPFIGVPTP
TLPENERLNEYADSFTNRFLNHYPFIHVTILKEYSLFKAILDENESTKNWENNQFYLENQRISIVCLPLL
VATIGAVLSNNKKDASNLYEASRRCIHVYLDSRKKIPTSLSANNNDSPLWLIQSLTLSVMYGLFADNDIS
LNVVIRQVNALNSLVKTSGLNRTSIIDLFNINKPLDNELWNQFVKIESTVRTIHTIFQISSNLSALYNIIPS
LKIDDLMITLPVPTTLWQADSFVKFKSLSYGNQIPFQYTRVLQNLIDYNQPLSDGKFLYENHVSEFGLIC
LQNGLHQYSYFQKLTAVNNREDALFTKVVNSLHSWDRMISNSDLFPKKIYQQSCLILDSKLLNNFLIVK
SSLKVSTGDVSSLNKLKENVWLKNWNQVCAIYYNSFMNIPAPSIQKKYNDIEFVDDMINLSLIIIKIMKLI
FYNNVKDNYEDENDFKLQELNLTFDNFDEKISLNLTILFDIFLMIYKIITNYEKFMKIKHKFNYYNSNSN
ISFLHHFELSSVINNTQMNQNDYMKTDIDEKLDQLFHIYQTFFRLYLDLEKFMKFKFNYHDFETEFSSL
SISNILNTHAASNNDTNAADAMNAKDEKISPTTLNSVLLADEGNENSGRNNDSDRLFMLNELINFEVG
LKFLKIGESFFDFLYENNYKFIHFKNLNDGMFHIRIYLENRLDGGV* (SEQ ID NO: 10)
>11 Mit1 
MSTAAPIKEESQFAHLTLMNKDIPSNAKQAKSKVSAAPAKTGSKSAGGSGNNNAAPVKKRVRTGCLTC
RKKHKKCDENRNPKCDFCTLKGLECVWPENNKKNIFVNNSMKDFLGKKTVDGADSLNLAVNLQQQQ
SSNTIANQSLSSIGLESFGYGSGIKNEFNFQDLIGSNSGSSDPTFSVDADEAQKLDISNKNSRKRQKLGLLP
VSNATSHLNGFNGMSNGKSHSFSSPSGTNDDELSGLMFNSPSFNPLTVNDSTNNSNHNIGLSPMSCLFS
TVQEASQKKHGNSSRHFSYPSGPEDLWFNEFQKQALTANGENAVQQGDDASKNNTAIPKDQSSNSSIF
SSRSSAASSNSGDDIGRMGPFSKGPEIEFNYDSFLESLKAESPSSSKYNLPETLKEYMTLSSSHLNSQHSD
TLANGTNGNYSSTVSNNLSLSLNSFSFSDKFSLSPPTITDAEKFSLMRNFIDNISPWFDTFDNTKQFGTK
IPVLAKKCSSLYYAILAISSRQRERIKKEHNEKTLQCYQYSLQQLIPTVQSSNNIEYIITCILLSVFHIMSSEP
STQRDIIVSLAKYIQACNINGFTSNDKLEKSIFWNYVNLDLATCAIGEESMVIPFSYWVKETTDYKTIQD
VKPFFTKKTSTTTDDDLDDMYAIYMLYISGRIINLLNCRDAKLNFEPKWEFLWNELNEWELNKPLTFQ
SIVQFKANDESQGGSTFPTVLFSNSRSCYSNQLYHMSYIILVQNKPRLYKIPFTTVSASMSSPSDNKAGM
SASSTPASDHHASGDHLSPRSVEPSLSTTLSPPPNANGAGNKFRSTLWHAKQICGISINNNHNSNLAAK
VNSLQPLWHAGKLISSKSEHTQLLKLLNNLECATGWPMNWKGKELIDYWNVEE* (SEQ ID NO: 11)
>12 Prm1 
MPPKHRLEQSIQPMASQQIVPGNKVILPNPKVDAKSTPNISVQKRRRVTRACDECRKKKVKCDGQQPCI
HCTVYSYECTYSQPSSKKRQGQSLSLSAPSNINATSSVQKSVKPPEIDFQRMRDALKYYEDLLNQLIYPN
SAPTVRVNPIRLASILKQLRADKSSDELISVKALSDNYIEMLHKTMQQPVQQPAPPSLGQGGSFSNHSPN
HNNASIDGSIESNLGREIRIILPPRDIALKLIYKTWDNACVLFRFYHRPAFIEDLNELYETDLANYTNKQQ
 
RFLPLVYSVMACGALFCKTDGINHGQKSSKPKDSSDESLIDDEGYKYFIAARKLIDITDTRDTYGIQTIVM
LIIFLQCSARLSTCYSYIGIALRAALREGLHRQLNYPFNPIELETRKRLFWTIYKMDIYVNTMLGLPRTISE
EDFDQEMPIELDDENISETGYRFDLQGTKLSSSGIANAHTRLIFIMKKIVKKLYPVKLQKPTSNSGDTPL
ENNDLLAHEIVHELEMDLQNWVNSLPAELKPGIEPPTEYFKANRLLHLAYLHVKIILYRPFIHYISEKDK
VGNSSIPPSPEEITSIEKAKNCVNVARIVVKLAEDMINRKMLSGSYWFSIYTIFFSVACLVYYVHFAPPKK
DNGELDPQYMEIKKDTESGREVLNILKDSSMAARRTYNILNSLFEQLNRRTAKVNLAKAQQPPSGLNN
PAATQYQKQGEHRQLQPSNYSGTVKSVDPENIDYSSFGSQFENTNIEDGSSNTKIDQKVNGVNYIDGVF
TGINLNMPNLSETSNTQGIDNPAFQSINNSNLNNNFVQTKYIPGMMDQLDMKIFGRFLPPYMLNSNKV
EQGQNERNLSGQPSSSNTPDGSQPVTVLDGLYPLQNDNNNNHDPGNSKSVVNNSNSVENLLQNFTMV
PSGLSSTVQNPEAAQKFNNHMSNISNMNDPRRASVATSDGSNDMDHHSQGPINKDLKPLSNYEFDDL
FFNDWTTAPDTINFDS* (SEQ ID NO: 12)
>13 Mpp1 
MSISRDDDHPRPKRARSGKPKKETPPAKRRVRTGCLTCRRKHKKCDENRPKCDFCTAKGLECVWPEE
VKKNVFVNNSFKDFCFSQSKHRDRFRKPTDFDLSSSLSPLDYHLGPDDPRTRKKAYSLDVADHYLSAF
DGYSRKNEYLDAPLTTAYPLLETSESPLSGLFNAPQELPSQLTRTPSFGASSLSYFDTLQSYSMNPSLSSG
SNDKISTASSVSSDMSLAKKDADFEHRYSFAEPQKKSKFGLDYYDLPNTLNDLMCLFIATKDSTSGSKLP
NNETIFEIFTDKSTMEAPSIDDHEMMLLMETYIDQIAHFLNKSDETQCNIFMTKIPELAHRFPSLKYAIL
GLASRHLEKVRSDYSGEKTLQYYTYSLQQLSMCLNSDKNTLEVVATCVLLCYFEILSTNPDSWHSRLEN
CGSMLKVCHINARSETELEKALFWSFAALDIGCCAVGDRKTVVRPEDWFGTDAPSSPTETGTALQSML
YLSCQIFQLVSSNDDQSEFDSCWKSLWKQLGDWERARSSNMRGFCFKRDNSFPETLFSSSTALCANQL
FHTCCILMLQTKPRLVKLHEPEKQERTPDTLFQSAIKTEQGESVSTSSLMSRSQIWHAKQIIGINVCNMK
SASSRSLGCQILSLQAIWIAGKLISSAHEHSIILQLLQRLESWSSMSMDWREKQLTEFWKREC* (SEQ ID
NO: 13)
>14 mMxr1- mMxr1AD 
MSNLPPTFGSTRQSPEDQSPPVPKELSFNGTTPSGKLRLFVCQTCTRAFARQEHLKRHERSHTKEKPFS
CGICSRKFSRRDLLLRHAQKLHSNCSDAAITRLRRKATRRSSNAAGSISGSTPVTTPNTMGTPEDGEKR
KVQKLAGRRDSNEQKLQLQQQHLQQQPLQYQQSLKQHENQVQQPDQDPLISPRMQLFNDSNHHVN
NLFDLGLRRASFSAVSGNNYAHYVNNFQQDASSTNPNQDSNNAEFENIEFSTPQMMPVEDAETWMN
NMGPIPNFSLDVNRNIGDSFTDIQHKNSEPIISEPPKDTAPNDKKLNGYSFYEAPIKPLESLFSVRNTKR
NKYKTNDDSPDTVDNNSAPAANTIQELESSLNASKNFCLPTGYSFYGNLDQQTFSNTLSCGGGGSTSDA
AITRLRRKATRRSSNAAGSISGSTPVTTPNTMGTPEDGEKRKVQKLAGRRDSNEQKLQLQQQHLQQQP
 
QLQYQQSLKQHENQVQQPDQDPLISPRMQLFNDSNHHVNNLFDLGLRRASFSAVSGNNYAHYVNNFQ
QDASSTNPNQDSNNAEFENIEFSTPQMMPVEDAETWMNNMGPIPNFSLDVNRNIGDSFTDIQHKNSE
PIISEPPKDTAPNDKKLNGYSFYEAPIKPLESLFSVRNTKRNKYKTNDDSPDTVDNNSAPAANTIQELES
SLNASKNFCLPTGYSFYGNLDQQTFSNTLSC* (SEQ ID NO: 14)
>15 Mxr1-Prm1AD 
MSNLPPTFGSTRQSPEDQSPPVPKELSFNGTTPSGKLRLFVCQTCTRAFARQEHLKRHERSHTKEKPFS
CGICSRKFSRRDLLLRHAQKLHSNCSDAAITRLRRKATRRSSNAAGSISGSTPVTTPNTMGTPEDGEKR
KVQKLAGRRDSNEQKLQLQQQHLQQQPQLQYQQSLKQHENQVQQPDQDPLISPRMQLFNDSNHHVN
NLFDLGLRRASFSAVSGNNYAHYVNNFQQDASSTNPNQDSNNAEFENIEFSTPQMMPVEDAETWMN
NMGPIPNFSLDVNRNIGDSFTDIQHKNSEPIISEPPKDTAPNDKKLNGYSFYEAPIKPLESLFSVRNTKR
NKYKTNDDSPDTVDNNSAPAANTIQELESSLNASKNFCLPTGYSFYGNLDQQTFSNTLSCTSSNATISPI
LLDNSINNNSTSDVRPEFRTQSVTSEMSQAPPPQKNNSKYSTEVLFTSNMRSFIHYALSKYPFIGVPTP
TLPENERLNEYADSFTNRFLNHYPFIHVTILKEYSLFKAILDENESTKNWENNQFYLENQRISIVCLPLL
VATIGAVLSNNKKDASNLYEASRRCIHVYLDSRKKIPTSLSANNNDSPLWLIQSLTLSVMYGLFADNDIS
LNVVIRQVNALNSLVKTSGLNRTSIIDLFNINKPLDNELWNQFVKIESTVRTIHTIFQISSNLSALYNIIPS
LKIDDLMITLPVPTTLWQADSFVKFKSLSYGNQIPFQYTRVLQNLIDYNQPLSDGKFLYENHVSEFGLIC
LQNGLHQYSYFQKLTAVNNREDALFTKVVNSLHSWDRMISNSDLFPKKIYQQSCLILDSKLLNNFLIVK
SSLKVSTGDVSSLNKLKENVWLKNWNQVCAIYYNSFMNIPAPSIQKKYNDIEFVDDMINLSLIIIKIMKLI
FYNNVKDNYEDENDFKLQELNLTFDNFDEKISLNLTILFDIFLMIYKIITNYEKFMKIKHKFNYYNSNSN
ISFLHHFELSSVINNTQMNQNDYMKTDIDEKLDQLFHIYQTFFRLYLDLEKFMKFKFNYHDFETEFSSL
SISNILNTHAASNNDTNAADAMNAKDEKISPTTLNSVLLADEGNENSGRNNDSDRLFMLNELINFEVG
LKFLKIGESFFDFLYENNYKFIHFKNLNDGMFHIRIYLENRLDGGVGGGGSGQSLSLSAPSNINATSSVQK
SVKPPEIDFQRMRDALKYYEDLLNQLIYPNSAPTVRVNPIRLASILKQLRADKSSDELISVKALSDNYIEM
LHKTMQQPVQQPAPPSLGQGGSFSNHSPNHNNASIDGSIESNLGREIRIILPPRDIALKLIYKTWDNACV
LFRFYHRPAFIEDLNELYETDLANYTNKQQRFLPLVYSVMACGALFCKTDGINHGQKSSKPKDSSDESLI
DDEGYKYFIAARKLIDITDTRDTYGIQTIVMLIIFLQCSARLSTCYSYIGIALRAALREGLHRQLNYPFNPIE
LETRKRLFWTIYKMDIYVNTMLGLPRTISEEDFDQEMPIELDDENISETGYRFDLQGTKLSSSGIANAHT
RLIFIMKKIVKKLYPVKLQKPTSNSGDTPLENNDLLAHEIVHELEMDLQNWVNSLPAELKPGIEPPTEY
FKANRLLHLAYLHVKIILYRPFIHYISEKDKVGNSSIPPSPEEITSIEKAKNCVNVARIVVKLAEDMINRK
MLSGSYWFSIYTIFFSVACLVYYVHFAPPKKDNGELDPQYMEIKKDTESGREVLNILKDSSMAARRTYNI
LNSLFEQLNRRTAKVNLAKAQQPPSGLNNPAATQYQKQGEHRQLQPSNYSGTVKSVDPENIDYSSFGS
QFENTNIEDGSSNTKIDQKVNGVNYIDGVFTGINLNMPNLSETSNTQGIDNPAFQSINNSNLNNNFVQT
 
KYIPGMMDQLDMKIFGRFLPPYMLNSNKVEQGQNERNLSGQPSSSNTPDGSQPVTVLDGLYPLQNDN
NNNHDPGNSKSVVNNSNSVENLLQNFTMVPSGLSSTVQNPEAAQKFNNHMSNISNMNDPRRASVATS
DGSNDMDHHSQGPINKDLKPLSNYEFDDLFFNDWTTAPDTINFDS* (SEQ ID NO: 15)
>16 Mxr1D-Mit1AD 
MSNLPPTFGSTRQSPEDQSPPVPKELSFNGTTPSGKLRLFVCQTCTRAFARQEHLKRHERSHTKEKPFS
CGICSRKFSRRDLLLRHAQKLHSNCSGGGGSVNNSMKDFLGKKTVDGADSLNLAVNLQQQQSSNTIAN
QSLSSIGLESFGYGSGIKNEFNFQDLIGSNSGSSDPTFSVDADEAQKLDISNKNSRKRQKLGLLPVSNATS
HLNGFNGMSNGKSHSFSSPSGTNDDELSGLMFNSPSFNPLTVNDSTNNSNHNIGLSPMSCLFSTVQEAS
QKKHGNSSRHFSYPSGPEDLWFNEFQKQALTANGENAVQQGDDASKNNTAIPKDQSSNSSIFSSRSSA
ASSNSGDDIGRMGPFSKGPEIEFNYDSFLESLKAESPSSSKYNLPETLKEYMTLSSSHLNSQHSDTLANG
TNGNYSSTVSNNLSLSLNSFSFSDKFSLSPPTITDAEKFSLMRNFIDNISPWFDTFDNTKQFGTKIPVLAK
KCSSLYYAILAISSRQRERIKKEHNEKTLQCYQYSLQQLIPTVQSSNNIEYIITCILLSVFHIMSSEPSTQRDI
IVSLAKYIQACNINGFTSNDKLEKSIFWNYVNLDLATCAIGEESMVIPFSYWVKETTDYKTIQDVKPFFT
KKTSTTTDDDLDDMYAIYMLYISGRIINLLNCRDAKLNFEPKWEFLWNELNEWELNKPLTFQSIVQFK
ANDESQGGSTFPTVLFSNSRSCYSNQLYHMSYIILVQNKPRLYKIPFTTVSASMSSPSDNKAGMSASSTP
ASDHHASGDHLSPRSVEPSLSTTLSPPPNANGAGNKFRSTLWHAKQICGISINNNHNSNLAAKVNSLQP
LWHAGKLISSKSEHTQLLKLLNNLECATGWPMNWKGKELIDYWNVEE* (SEQ ID NO: 16)
>17 Mxr1D-Prm1AD 
MSNLPPTFGSTRQSPEDQSPPVPKELSFNGTTPSGKLRLFVCQTCTRAFARQEHLKRHERSHTKEKPFS
CGICSRKFSRRDLLLRHAQKLHSNCSGGGGSGQSLSLSAPSNINATSSVQKSVKPPEIDFQRMRDALKYY
EDLLNQLIYPNSAPTVRVNPIRLASILKQLRADKSSDELISVKALSDNYIEMLHKTMQQPVQQPAPPSLG
QGGSFSNHSPNHNNASIDGSIESNLGREIRIILPPRDIALKLIYKTWDNACVLFRFYHRPAFIEDLNELYE
TDLANYTNKQQRFLPLVYSVMACGALFCKTDGINHGQKSSKPKDSSDESLIDDEGYKYFIAARKLIDITD
TRDTYGIQTIVMLIIFLQCSARLSTCYSYIGIALRAALREGLHRQLNYPFNPIELETRKRLFWTIYKMDIYV
NTMLGLPRTISEEDFDQEMPIELDDENISETGYRFDLQGTKLSSSGIANAHTRLIFIMKKIVKKLYPVKLQ
KPTSNSGDTPLENNDLLAHEIVHELEMDLQNWVNSLPAELKPGIEPPTEYFKANRLLHLAYLHVKIILY
RPFIHYISEKDKVGNSSIPPSPEEITSIEKAKNCVNVARIVVKLAEDMINRKMLSGSYWFSIYTIFFSVACL
VYYVHFAPPKKDNGELDPQYMEIKKDTESGREVLNILKDSSMAARRTYNILNSLFEQLNRRTAKVNLA
KAQQPPSGLNNPAATQYQKQGEHRQLQPSNYSGTVKSVDPENIDYSSFGSQFENTNIEDGSSNTKIDQK
VNGVNYIDGVFTGINLNMPNLSETSNTQGIDNPAFQSINNSNLNNNFVQTKYIPGMMDQLDMKIFGRF
LPPYMLNSNKVEQGQNERNLSGQPSSSNTPDGSQPVTVLDGLYPLQNDNNNNHDPGNSKSVVNNSNS
 
VENLLQNFTMVPSGLSSTVQNPEAAQKFNNHMSNISNMNDPRRASVATSDGSNDMDHHSQGPINKDL
KPLSNYEFDDLFFNDWTTAPDTINFDS* (SEQ ID NO: 17)
>18 Mit1-mMxr1AD 
MSTAAPIKEESQFAHLTLMNKDIPSNAKQAKSKVSAAPAKTGSKSAGGSGNNNAAPVKKRVRTGCLTC
RKKHKKCDENRNPKCDFCTLKGLECVWPENNKKNIFVNNSMKDFLGKKTVDGADSLNLAVNLQQQQ
SSNTIANQSLSSIGLESFGYGSGIKNEFNFQDLIGSNSGSSDPTFSVDADEAQKLDISNKNSRKRQKLGLLP
VSNATSHLNGFNGMSNGKSHSFSSPSGTNDDELSGLMFNSPSFNPLTVNDSTNNSNHNIGLSPMSCLFS
TVQEASQKKHGNSSRHFSYPSGPEDLWFNEFQKQALTANGENAVQQGDDASKNNTAIPKDQSSNSSIF
SSRSSAASSNSGDDIGRMGPFSKGPEIEFNYDSFLESLKAESPSSSKYNLPETLKEYMTLSSSHLNSQHSD
TLANGTNGNYSSTVSNNLSLSLNSFSFSDKFSLSPPTITDAEKFSLMRNFIDNISPWFDTFDNTKQFGTK
IPVLAKKCSSLYYAILAISSRQRERIKKEHNEKTLQCYQYSLQQLIPTVQSSNNIEYIITCILLSVFHIMSSEP
STQRDIIVSLAKYIQACNINGFTSNDKLEKSIFWNYVNLDLATCAIGEESMVIPFSYWVKETTDYKTIQD
VKPFFTKKTSTTTDDDLDDMYAIYMLYISGRIINLLNCRDAKLNFEPKWEFLWNELNEWELNKPLTFQ
SIVQFKANDESQGGSTFPTVLFSNSRSCYSNQLYHMSYIILVQNKPRLYKIPFTTVSASMSSPSDNKAGM
SASSTPASDHHASGDHLSPRSVEPSLSTTLSPPPNANGAGNKFRSTLWHAKQICGISINNNHNSNLAAK
VNSLQPLWHAGKLISSKSEHTQLLKLLNNLECATGWPMNWKGKELIDYWNVEEGGGGSDAAITRLRR
KATRRSSNAAGSISGSTPVTTPNTMGTPEDGEKRKVQKLAGRRDSNEQKLQLQQQHLQQQPQLQYQQ
SLKQHENQVQQPDQDPLISPRMQLFNDSNHHVNNLFDLGLRRASFSAVSGNNYAHYVNNFQQDASST
NPNQDSNNAEFENIEFSTPQMMPVEDAETWMNNMGPIPNFSLDVNRNIGDSFTDIQHKNSEPIISEPP
KDTAPNDKKLNGYSFYEAPIKPLESLFSVRNTKRNKYKTNDDSPDTVDNNSAPAANTIQELESSLNASK
NFCLPTGYSFYGNLDQQTFSNTLSC* (SEQ ID NO: 18)
>19 Mit1D-mMxr1AD 
MSTAAPIKEESQFAHLTLMNKDIPSNAKQAKSKVSAAPAKTGSKSAGGSGNNNAAPVKKRVRTGCLTC
RKKHKKCDENRNPKCDFCTLKGLECVWPENNKKNIFGGGGSSNCSDAAITRLRRKATRRSSNAAGSIS
GSTPVTTPNTMGTPEDGEKRKVQKLAGRRDSNEQKLQLQQQHLQQQPQLQYQQSLKQHENQVQQPD
QDPLISPRMQLFNDSNHHVNNLFDLGLRRASFSAVSGNNYAHYVNNFQQDASSTNPNQDSNNAEFENI
EFSTPQMMPVEDAETWMNNMGPIPNFSLDVNRNIGDSFTDIQHKNSEPIISEPPKDTAPNDKKLNGYS
FYEAPIKPLESLFSVRNTKRNKYKTNDDSPDTVDNNSAPAANTIQELESSLNASKNFCLPTGYSFYGNL
DQQTFSNTLSC* (SEQ ID NO: 19)
>20 Mit1D-Mxr1AD 
MSTAAPIKEESQFAHLTLMNKDIPSNAKQAKSKVSAAPAKTGSKSAGGSGNNNAAPVKKRVRTGCLTC
RKKHKKCDENRNPKCDFCTLKGLECVWPENNKKNIFGGGGSSNCSDAAITRLRRKATRRSSNAAGSIS
GSTPVTTPNTMGTPEDGEKRKVQKLAGRRDSNEQKLQLQQQHLQQQPQLQYQQSLKQHENQVQQPD
QDPLISPRMQLFNDSNHHVNNLFDLGLRRASFSAVSGNNYAHYVNNFQQDASSTNPNQDSNNAEFENI
EFSTPQMMPVEDAETWMNNMGPIPNFSLDVNRNIGDSFTDIQHKNSEPIISEPPKDTAPNDKKLNGYS
FYEAPIKPLESLFSVRNTKRNKYKTNDDSPDTVDNNSAPAANTIQELESSLNASKNFCLPTGYSFYGNL
DQQTFSNTLSCTSSNATISPILLDNSINNNSTSDVRPEFRTQSVTSEMSQAPPPPQKNNSKYSTEVLFTS
NMRSFIHYALSKYPFIGVPTPTLPENERLNEYADSFTNRFLNHYPFIHVTILKEYSLFKAILDENESTKN
WENNQFYLENQRISIVCLPLLVATIGAVLSNNKKDASNLYEASRRCIHVYLDSRKKIPTSLSANNNDSPL
WLIQSLTLSVMYGLFADNDISLNVVIRQVNALNSLVKTSGLNRTSIIDLFNINKPLDNELWNQFVKIEST
VRTIHTIFQISSNLSALYNIIPSLKIDDLMITLPVPTTLWQADSFVKFKSLSYGNQIPFQYTRVLQNLIDYN
QPLSDGKFLYENHVSEFGLICLQNGLHQYSYFQKLTAVNNREDALFTKVVNSLHSWDRMISNSDLFPK
KIYQQSCLILDSKLLNNFLIVKSSLKVSTGDVSSLNKLKENVWLKNWNQVCAIYYNSFMNIPAPSIQKKY
NDIEFVDDMINLSLIIIKIMKLIFYNNVKDNYEDENDFKLQELNLTFDNFDEKISLNLTILFDIFLMIYKIIT
NYEKFMKIKHKFNYYNSNSNISFLHHFELSSVINNTQMNQNDYMKTDIDEKLDQLFHIYQTFFRLYLDL
EKFMKFKFNYHDFETEFSSLSISNILNTHAASNNDTNAADAMNAKDEKISPTTLNSVLLADEGNENSG
RNNDSDRLFMLNELINFEVGLKFLKIGESFFDFLYENNYKFIHFKNLNDGMFHIRIYLENRLDGGV*
(SEQ ID NO: 20)
>21 Mit1D-Prm1AD 
MSTAAPIKEESQFAHLTLMNKDIPSNAKQAKSKVSAAPAKTGSKSAGGSGNNNAAPVKKRVRTGCLTC
RKKHKKCDENRNPKCDFCTLKGLECVWPENNKKNIFGGGGSGQSLSLSAPSNINATSSVQKSVKPPEID
FQRMRDALKYYEDLLNQLIYPNSAPTVRVNPIRLASILKQLRADKSSDELISVKALSDNYIEMLHKTMQ
QPVQQPAPPSLGQGGSFSNHSPNHNNASIDGSIESNLGREIRIILPPRDIALKLIYKTWDNACVLFRFYHR
PAFIEDLNELYETDLANYTNKQQRFLPLVYSVMACGALFCKTDGINHGQKSSKPKDSSDESLIDDEGYK
YFIAARKLIDITDTRDTYGIQTIVMLIIFLQCSARLSTCYSYIGIALRAALREGLHRQLNYPFNPIELETRKR
LFWTIYKMDIYVNTMLGLPRTISEEDFDQEMPIELDDENISETGYRFDLQGTKLSSSGIANAHTRLIFIM
KKIVKKLYPVKLQKPTSNSGDTPLENNDLLAHEIVHELEMDLQNWVNSLPAELKPGIEPPTEYFKANR
LLHLAYLHVKIILYRPFIHYISEKDKVGNSSIPPSPEEITSIEKAKNCVNVARIVVKLAEDMINRKMLSGSY
WFSIYTIFFSVACLVYYVHFAPPKKDNGELDPQYMEIKKDTESGREVLNILKDSSMAARRTYNILNSLFE
QLNRRTAKVNLAKAQQPPSGLNNPAATQYQKQGEHRQLQPSNYSGTVKSVDPENIDYSSFGSQFENTN
IEDGSSNTKIDQKVNGVNYIDGVFTGINLNMPNLSETSNTQGIDNPAFQSINNSNLNNNFVQTKYIPGM
MDQLDMKIFGRFLPPYMLNSNKVEQGQNERNLSGQPSSSNTPDGSQPVTVLDGLYPLQNDNNNNHDP
 
GNSKSVVNNSNSVENLLQNFTMVPSGLSSTVQNPEAAQKFNNHMSNISNMNDPRRASVATSDGSNDM
DHHSQGPINKDLKPLSNYEFDDLFFNDWTTAPDTINFDS* (SEQ ID NO: 21)
>22 Prm1-mMxr1AD 
MPPKHRLEQSIQPMASQQIVPGNKVILPNPKVDAKSTPNISVQKRRRVTRACDECRKKKVKCDGQQPCI
HCTVYSYECTYSQPSSKKRQGQSLSLSAPSNINATSSVQKSVKPPEIDFQRMRDALKYYEDLLNQLIYPN
SAPTVRVNPIRLASILKQLRADKSSDELISVKALSDNYIEMLHKTMQQPVQQPAPPSLGQGGSFSNHSPN
HNNASIDGSIESNLGREIRIILPPRDIALKLIYKTWDNACVLFRFYHRPAFIEDLNELYETDLANYTNKQQ
RFLPLVYSVMACGALFCKTDGINHGQKSSKPKDSSDESLIDDEGYKYFIAARKLIDITDTRDTYGIQTIVM
LIIFLQCSARLSTCYSYIGIALRAALREGLHRQLNYPFNPIELETRKRLFWTIYKMDIYVNTMLGLPRTISE
EDFDQEMPIELDDENISETGYRFDLQGTKLSSSGIANAHTRLIFIMKKIVKKLYPVKLQKPTSNSGDTPL
ENNDLLAHEIVHELEMDLQNWVNSLPAELKPGIEPPTEYFKANRLLHLAYLHVKIILYRPFIHYISEKDK
VGNSSIPPSPEEITSIEKAKNCVNVARIVVKLAEDMINRKMLSGSYWFSIYTIFFSVACLVYYVHFAPPKK
DNGELDPQYMEIKKDTESGREVLNILKDSSMAARRTYNILNSLFEQLNRRTAKVNLAKAQQPPSGLNN
PAATQYQKQGEHRQLQPSNYSGTVKSVDPENIDYSSFGSQFENTNIEDGSSNTKIDQKVNGVNYIDGVF
TGINLNMPNLSETSNTQGIDNPAFQSINNSNLNNNFVQTKYIPGMMDQLDMKIFGRFLPPYMLNSNKV
EQGQNERNLSGQPSSSNTPDGSQPVTVLDGLYPLQNDNNNNHDPGNSKSVVNNSNSVENLLQNFTMV
PSGLSSTVQNPEAAQKFNNHMSNISNMNDPRRASVATSDGSNDMDHHSQGPINKDLKPLSNYEFDDL
FFNDWTTAPDTINFDSGGGGSDAAITRLRRKATRRSSNAAGSISGSTPVTTPNTMGTPEDGEKRKVQK
LAGRRDSNEQKLQLQQQHLQQQPQLQYQQSLKQHENQVQQPDQDPLISPRMQLFNDSNHHVNNLFD
LGLRRASFSAVSGNNYAHYVNNFQQDASSTNPNQDSNNAEFENIEFSTPQMMPVEDAETWMNNMGP
IPNFSLDVNRNIGDSFTDIQHKNSEPIISEPPKDTAPNDKKLNGYSFYEAPIKPLESLFSVRNTKRNKYKT
NDDSPDTVDNNSAPAANTIQELESSLNASKNFCLPTGYSFYGNLDQQTFSNTLSC* (SEQ ID NO: 22)
>23 Prm1D-mMxr1AD 
MPPKHRLEQSIQPMASQQIVPGNKVILPNPKVDAKSTPNISVQKRRRVTRACDECRKKKVKCDGQQPCI
HCTVYSYECTYSQPSSKKRQGGGGSSNCSDAAITRLRRKATRRSSNAAGSISGSTPVTTPNTMGTPEDGE
KRKVQKLAGRRDSNEQKLQLQQQHLQQQPQLQYQQSLKQHENQVQQPDQDPLISPRMQLFNDSNHH
VNNLFDLGLRRASFSAVSGNNYAHYVNNFQQDASSTNPNQDSNNAEFENIEFSTPQMMPVEDAETW
MNNMGPIPNFSLDVNRNIGDSFTDIQHKNSEPIISEPPKDTAPNDKKLNGYSFYEAPIKPLESLFSVRNT
KRNKYKTNDDSPDTVDNNSAPAANTIQELESSLNASKNFCLPTGYSFYGNLDQQTFSNTLSC* (SEQ ID
NO: 23)
>24 Prm1D-Mxr1AD 
MPPKHRLEQSIQPMASQQIVPGNKVILPNPKVDAKSTPNISVQKRRRVTRACDECRKKKVKCDGQQPCI
HCTVYSYECTYSQPSSKKRQGGGGSSNCSDAAITRLRRKATRRSSNAAGSISGSTPVTTPNTMGTPEDGE
KRKVQKLAGRRDSNEQKLQLQQQHLQQQPQLQYQQSLKQHENQVQQPDQDPLISPRMQLFNDSNHH
VNNLFDLGLRRASFSAVSGNNYAHYVNNFQQDASSTNPNQDSNNAEFENIEFSTPQMMPVEDAETW
MNNMGPIPNFSLDVNRNIGDSFTDIQHKNSEPIISEPPKDTAPNDKKLNGYSFYEAPIKPLESLFSVRNT
KRNKYKTNDDSPDTVDNNSAPAANTIQELESSLNASKNFCLPTGYSFYGNLDQQTFSNTLSCTSSNATI
SPILLDNSINNNSTSDVRPEFRTQSVTSEMSQAPPPPQKNNSKYSTEVLFTSNMRSFIHYALSKYPFIGVP
TPTLPENERLNEYADSFTNRFLNHYPFIHVTILKEYSLFKAILDENESTKNWENNQFYLENQRISIVCLP
LLVATIGAVLSNNKKDASNLYEASRRCIHVYLDSRKKIPTSLSANNNDSPLWLIQSLTLSVMYGLFADND
ISLNVVIRQVNALNSLVKTSGLNRTSIIDLFNINKPLDNELWNQFVKIESTVRTIHTIFQISSNLSALYNIIP
SLKIDDLMITLPVPTTLWQADSFVKFKSLSYGNQIPFQYTRVLQNLIDYNQPLSDGKFLYENHVSEFGLI
CLQNGLHQYSYFQKLTAVNNREDALFTKVVNSLHSWDRMISNSDLFPKKIYQQSCLILDSKLLNNFLIV
KSSLKVSTGDVSSLNKLKENVWLKNWNQVCAIYYNSFMNIPAPSIQKKYNDIEFVDDMINLSLIIIKIMK
LIFYNNVKDNYEDENDFKLQELNLTFDNFDEKISLNLTILFDIFLMIYKIITNYEKFMKIKHKFNYYNSNS
NISFLHHFELSSVINNTQMNQNDYMKTDIDEKLDQLFHIYQTFFRLYLDLEKFMKFKFNYHDFETEFSS
LSISNILNTHAASNNDTNAADAMNAKDEKISPTTLNSVLLADEGNENSGRNNDSDRLFMLNELINFEV
GLKFLKIGESFFDFLYENNYKFIHFKNLNDGMFHIRIYLENRLDGGV* (SEQ ID NO: 24)
>25 Prm1D-Mit1AD 
MPPKHRLEQSIQPMASQQIVPGNKVILPNPKVDAKSTPNISVQKRRRVTRACDECRKKKVKCDGQQPCI
HCTVYSYECTYSQPSSKKRQGGGGSVNNSMKDFLGKKTVDGADSLNLAVNLQQQQSSNTIANQSLSSIG
LESFGYGSGIKNEFNFQDLIGSNSGSSDPTFSVDADEAQKLDISNKNSRKRQKLGLLPVSNATSHLNGFN
GMSNGKSHSFSSPSGTNDDELSGLMFNSPSFNPLTVNDSTNNSNHNIGLSPMSCLFSTVQEASQKKHG
NSSRHFSYPSGPEDLWFNEFQKQALTANGENAVQQGDDASKNNTAIPKDQSSNSSIFSSRSSAASSNSG
DDIGRMGPFSKGPEIEFNYDSFLESLKAESPSSSKYNLPETLKEYMTLSSSHLNSQHSDTLANGTNGNYS
STVSNNLSLSLNSFSFSDKFSLSPPTITDAEKFSLMRNFIDNISPWFDTFDNTKQFGTKIPVLAKKCSSLY
YAILAISSRQRERIKKEHNEKTLQCYQYSLQQLIPTVQSSNNIEYIITCILLSVFHIMSSEPSTQRDIIVSLAK
YIQACNINGFTSNDKLEKSIFWNYVNLDLATCAIGEESMVIPFSYWVKETTDYKTIQDVKPFFTKKTSTT
TDDDLDDMYAIYMLYISGRIINLLNCRDAKLNFEPKWEFLWNELNEWELNKPLTFQSIVQFKANDESQ
GGSTFPTVLFSNSRSCYSNQLYHMSYIILVQNKPRLYKIPFTTVSASMSSPSDNKAGMSASSTPASDHHA
SGDHLSPRSVEPSLSTTLSPPPNANGAGNKFRSTLWHAKQICGISINNNHNSNLAAKVNSLQPLWHAG
KLISSKSEHTQLLKLLNNLECATGWPMNWKGKELIDYWNVEE* (SEQ ID NO: 25)
>26 BtMb 
MGLSDGEWQLVLNAWGKVEADVAGHGQEVLIRLFTGHPETLEKFDKFKHLKTEAEMKASEDLKKHG
NTVLTALGGILKKKGHHEAEVKHLAESHANKHKIPVKYLEFISDAIIHVLHAKHPSDFGADAQAAMSKA
LELFRNDMAAQYKVLGFHG* (SEQ ID NO: 26)
>27
   (GGGGS)ₙ (SEQ ID NO: 27)
>28
   GSAGSAAGSGEF (SEQ ID NO: 28)
>29
   KESGSVSSEQLAQFRSLD (SEQ ID NO: 29)
>30
   EGKSSGSGSESKST (SEQ ID NO: 30)
>31
   (EAAAK)ₙ (SEQ ID NO: 31)

## Claims

1. A chimeric transcriptional activator, comprising at least a first portion and a second portion, wherein:
1) the first portion comprises:
i) a DNA binding domain; or
ii) a DNA binding domain and a first transcription activation domain; and
2) the second portion comprises at least one second transcription activation domain.

2. The chimeric transcriptional activator of claim 1, wherein the C-terminal end of the first portion is connected to the N-terminal end of the second portion directly or indirectly via a linker molecule.

3. The chimeric transcriptional activator of claim 1 or 2, wherein the DNA binding domain and the second transcription activation domain are derived from different transcriptional activators.

4. The chimeric transcriptional activator of any one of claims 1-3, wherein the DNA binding domain and the first transcription activation domain are derived from the same transcriptional activator.

5. The chimeric transcriptional activator of any one of claims 1-4, wherein the DNA binding domain is derived from transcriptional activator Mxr1, Mit1, Prm1, Adr1, Trm1, Trm2, Mpp1, or fragments or mutants thereof, preferably from transcriptional activator Mxr1, Mit1, Prm1, or fragments or mutants thereof.

6. The chimeric transcriptional activator of any one of claims 1-5, wherein the first transcription activation domain is derived from transcriptional activator Mxr1, Mit1, Prm1, Adr1, Trm1, Trm2, Mpp1, or fragments or mutants thereof, preferably from transcriptional activator Mxr1, Mit1, Prm1, or fragments or mutants thereof.

7. The chimeric transcriptional activator of any one of claims 1-6, wherein the transcriptional activator Mxr1 comprises a sequence set forth in SEQ ID NO: 10; the transcriptional activator Mit1 comprises a sequence set forth in SEQ ID NO: 11; and the transcriptional activator Prm1 comprises a sequence set forth in SEQ ID NO: 12.

8. The chimeric transcriptional activator of any one of claims 1-7, wherein the DNA binding domain comprises amino acids at positions 1-91 of the transcriptional activator Mxr1, amino acids at positions 1-104 of the transcriptional activator Mit1, or amino acids at positions 1-89 of the transcriptional activator Prm1.

9. The chimeric transcriptional activator of any one of claims 1-8, wherein the DNA binding domain has at least 50%, 60%, 70%, 80%, 90%, 92%, 95%, 98%, or 99% sequence identity to amino acids at positions 1-91 of the transcriptional activator Mxr1, amino acids at positions 1-104 of the transcriptional activator Mit1, or amino acids at positions 1-89 of the transcriptional activator Prm1.

10. The chimeric transcriptional activator of any one of claims 1-9, wherein the first transcription activation domain comprises amino acids at positions 92-1155 of the transcriptional activator Mxr1, amino acids at positions 92-400 of the transcriptional activator Mxr1, amino acids at positions 105-888 of the transcriptional activator Mit1, or amino acids at positions 90-989 of the transcriptional activator Prm1.

11. The chimeric transcriptional activator of any one of claims 1-10, wherein the first transcription activation domain has at least 50%, 60%, 70%, 80%, 90%, 92%, 95%, 98%, or 99% sequence identity to amino acids at positions 92-1155 of the transcriptional activator Mxr1, amino acids at positions 92-400 of the transcriptional activator Mxr1, amino acids at positions 105-888 of the transcriptional activator Mit1, or amino acids at positions 90-989 of the transcriptional activator Prm1.

12. The chimeric transcriptional activator of any one of claims 1-11, wherein the first portion comprises the transcriptional activator Mxr1, amino acids at positions 1-400 of the transcriptional activator Mxr1, amino acids at positions 1-91 of the transcriptional activator Mxr1, the transcriptional activator Mit1, amino acids at positions 1-104 of the transcriptional activator Mit1, the transcriptional activator Prm1, or amino acids at positions 1-89 of the transcriptional activator Prm1.

13. The chimeric transcriptional activator of any one of claims 1-12, wherein the second transcription activation domain is derived from transcriptional activator Mxr1, Mit1, Prm1, Adr1, Trm1, Trm2, Mpp1, or fragments or mutants thereof, preferably from transcriptional activator Mxr1, Mit1, Prm1, or fragments or mutants thereof.

14. The chimeric transcriptional activator of any one of claims 1-13, wherein the second transcription activation domain comprises amino acids at positions 92-1155 of the transcriptional activator Mxr1, amino acids at positions 92-400 of a truncated transcriptional activator mMxr1, amino acids at positions 105-888 of the transcriptional activator Mit1, or amino acids at positions 90-989 of the transcriptional activator Prm1.

15. The chimeric transcriptional activator of any one of claims 1-14, wherein the second transcription activation domain has at least 50%, 60%, 70%, 80%, 90%, 92%, 95%, 98%, or 99% sequence identity to amino acids at positions 92-1155 of the transcriptional activator Mxr1, amino acids at positions 92-400 of a truncated transcriptional activator mMxr1, amino acids at positions 105-888 of the transcriptional activator Mit1, or amino acids at positions 90-989 of the transcriptional activator Prm1.

16. The chimeric transcriptional activator of any one of claims 1-15, wherein the second portion comprises amino acids at positions 92-1155 of the transcriptional activator Mxr1, amino acids at positions 92-400 of a truncated transcriptional activator mMxr1, amino acids at positions 105-888 of the transcriptional activator Mit1, or amino acids at positions 90-989 of the transcriptional activator Prm1.

17. The chimeric transcriptional activator of any one of claims 1-16, comprising a sequence set forth in any one of SEQ ID NOs: 14-25.

18. An isolated nucleic acid molecule, comprising a coding nucleic acid sequence for the chimeric transcriptional activator of any one of claims 1-17.

19. An expression vector, comprising the nucleic acid molecule of claim 18.

20. The expression vector of claim 19, wherein in the expression vector, the coding nucleic acid sequence is operably connected to a methanol-inducible promoter.

21. The expression vector of claim 20, wherein the methanol-inducible promoter includes AOX1, DAS1, DAS2, CAT1, and PMP20 promoters, or mutants thereof, preferably AOX1 promoter.

22. A host cell, comprising the nucleic acid molecule of claim 18 or the expression vector of any one of claims 19-21.

23. A host cell expressing the chimeric transcriptional activator of any one of claims 1-17.

24. The host cell of claim 22 or 23, further comprising a nucleic acid sequence encoding a heterologous polypeptide that is operably connected to a methanol-inducible promoter.

25. The host cell of claim 24, wherein the heterologous polypeptide is derived from the PF00042 globulin family.

26. The host cell of claim 25, wherein the heterologous polypeptide is hemoglobin, preferably soy leghemoglobin LegH or bovine myoglobin.

27. The host cell of any one of claims 22-26, wherein the heterologous polypeptide comprises a sequence set forth in SEQ ID NO: 9 or SEQ ID NO: 26.

28. The host cell of any one of claims 22-27, further comprising a nucleic acid encoding at least one polypeptide involved in a heme biosynthesis pathway that is operably connected to the methanol-inducible promoter.

29. The host cell of any one of claims 22-28, wherein the host cell is a yeast cell, preferably *Pichia pastoris,* more preferably *Pichia pastoris* X-33 strain.

30. A method for preparing a protein of interest, comprising culturing the host cell of any one of claims 24-29 and isolating the protein of interest.

31. The method of claim 30, wherein the host cell is *Pichia pastoris,* and the process of culturing the host cell comprises adding methanol to a culture solution.

32. The method of claim 30 or 31, wherein the protein of interest is a protein from the PF00042 globulin family.

33. The method of any one of claims 30-32, wherein the protein of interest is hemoglobin, preferably soy leghemoglobin LegH or bovine myoglobin.
